Europäisches Patentamt

European Patent Office

Office européen des brevets

(11) Publication number: **0 302 387**
A2

## (12) EUROPEAN PATENT APPLICATION

(21) Application number: 88112189.1

(51) Int. Cl.⁴: **C12M 1/32**

(22) Date of filing: 28.07.88

(30) Priority: 06.08.87 IT 8343087

(43) Date of publication of application:
08.02.89 Bulletin 89/06

(84) Designated Contracting States:
AT BE CH DE ES FR GB GR LI LU NL SE

(71) Applicant: S.I.R.E. di De Monte R., Duic G.B. &
C. s.a.s.
via L. Menis n.3/2
I-33011 Artegna (UD)(IT)

(72) Inventor: **Enzo, Breda**
via Gorizia n. 86
I-33100 Udine (UD)(IT)
Inventor: **Alessandra, Petri**
via Roma n. 14/4
I-33010 Magnano in Riviera (UD)(IT)

(54) **Device suitable to withdraw and distribute automatically analitical samples into plates containing culture media for microbiology.**

(57) The proposed device allows to perform all operations concerning the microbiological analysis automatically without intervention of the operator and in particular :
- collection and withdrawal of samples;
- distribution of material into the Petri plates containing the culture media;
- sterilisation of the loop used to withdraw and distribute the samples.
This is obtained by the device composed by :
- means to collect, withdraw and distribute the samples to be analysed including the device for the direct contact with the samples;
- means for positioning the samples; - means for positioning the plates;
- means for opening the plates and for their successive closing;
- means to position the plates;
- means to allow the motion of the above-mentioned means;
- means to provide the sterilisation of the device which is in contact with the samples;
- means to control all operations and interface with operator.

EP 0 302 387 A2

**Device suitable to withdraw and distribute automatically analitical samples into plates containing culture media for microbiology.**

The object of the invention is a device that can automatically collect and distribute analitical samples on plates containing culture media for microbiology.

STATE OF THE ART IN PREPARATION OF MICROBIAL CULTURES.

At the present time the microbiological analysis are routinely performed on a great amount and variety of materials which may be analysed by manual methods to obtain cultures of bacteria into Petri plates containing culture media wich allow the growth of bacteria. After incubation at controlled temperature, these cultures allow insulation, characterisation and identification of bacteria by using some biochemical reactions caracteristic for each group of microorganism.

These operations for obtaining the final results may be described as follow : the technician collects with calibrated loops the material to be analysed and streaks the medium contained into the plates; in this way, by effect of this dilution it is possible to obtain insulated colonies of bacteria wich may be identified by operator.

DISADVANTAGES OF THE PROCEDURE:

- The distribution phase of the material may represent, for the operator, a risk of contamination with pathological bacteria.
- A great variability of results is obtained depending on operator's procedures wich may vary with operator's skilfulness.
In this way it may be necessary a second distribution to obtain insulated colonies.
- A loss of time for all operations described with the consequence of a major cost for single analyses.

At the present time there exist some apparatus which tend to decrease the manual load in these analysis but all require the presence of the operator to execute the first step of analysis (the addition of material to be analysed) and only after this step, these apparatus perform the distribution on the plate.

Some extremely sophisticated apparatus perform automatically the identification of bacteria by using special multicuvette system and photometrical measure of turbidity of the grown colonies. These methodologies are very expensive and do not allow the microbiologist having insight into the morphological aspect of the microorganism or obtaining insulated colonies to select the study further.

The philosophical trend of microbiologist seems to be oriented on the identification of bacteria through the insulation and biochemical characterisation of these bacteria, and the insulating procedure is the milestone of this work.

ADVANTAGES OF THE PROPOSED DEVICE

The limitations and the disadvantages of the manual procedures may be avoided by the proposed device, wich has been studied for simplifying and standardizing the methodology and for preserving the philosophy of microbiological work.

The proposed device allows to perform all operations concerning the microbiological analysis automatically without intervention of the operator and in particular :
- collection and withdrawal of samples;
- distribution of material into the Petri plates containing the culture media;
- sterilisation of the loop used to withdraw and distribute the samples.

The proposed device allows to obtain:
- increased security for manipulating samples containing possible pathological bacteria;
- standardisation of the procedure in the laboratory indipendently of the operator running this work;
- saving time for operator who, otherwise, will be occupied with repetitive work;
- compatibility of this system with the philosophy of the microbiological laboratory which doesn't need any change of the operations already in use.

THE PROPOSED DEVICE IS COMPOSED BY :

- means to collect, withdraw and distribute the samples to be analysed including the device for the direct contact with the samples;
- means for positionig the samples housed into suitable containers;
- means for positioning the plates containing the growth media;
- means for opening the plates and for their successive closing;
- means to position the plates;
- means to allow the motion of the above-mentioned means;
- means to provide the sterilisation of the device which is in contact with the samples;
- means to control all operations;
- means of interface with operator.

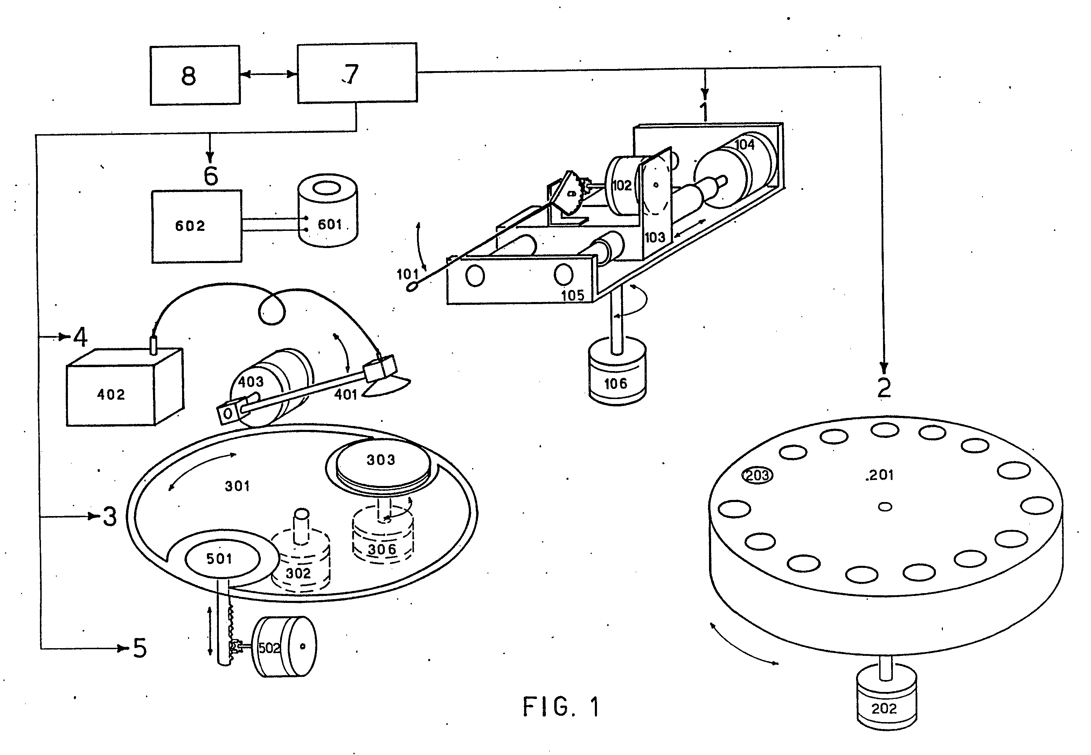

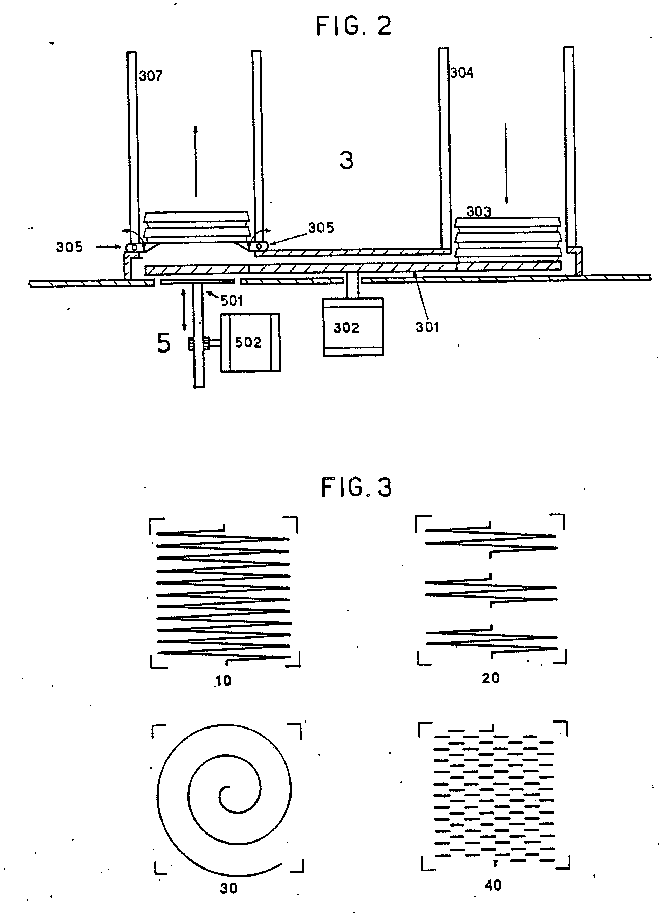

Here is described, as a simplified and non restrictive example, a particular realisation of the device with reference to fig.1, of inclosed tables, which represents a perspective view and fig.2 which represents a section of a part.

In fig.1 may be distinguished : (1) a mobile head which is formed by a three degrees of freedom robot; (101) a calibrated loop (of ferromagnetic material) suitable to contact the samples to be analysed; (102) a motor which, connected through a compound lever to the loop (101), allows the raising and the lowering of the loop (101); a sliding support (103) capable to hold the loop (101) and integral with the motor (102); a motor (104) which allows the advancement and withdrawal of the sliding support (103); a rotating support (105), integral with motor (104), which contains the sliding support (103); a motor (106) whose axis is connected to support (105) to allow the rotation; a support (2) for the samples composed of a rotating disk (201), which holds the samples (203), and of a motor (202) which allows the rotation of the rotating disk (201); a holder (3) for the culture plates composed of a distributor disk (301), of a motor (302) for the rotation of the distributor disk (301), of a motor (306) for the eventual rotation of the plates (303); a device (4) allowing the opening of the plates through a lever with a sucking disk (401) operated by a motor (403) and a vacuum pump (402) connected to the lever (401); (5) an apparatus to position the inoculated plates in which the position with a rod rack (501) is raised and lowered by a motor with a pinion (502); (6) an induction oven composed by a load coil (601) and an electronic circuit (602); (7) a microcomputer with the power electronic circuits for the control of functions and driving of motors; (8) a control unit for the acquisition of orders and for the interaction with the operator.

In fig.2 it may be distinguished : (3) an overview of the plates distributor; (302) a motor for the rotation of the distribution disk (301); (304) the holder to house the culture plates (303) before inoculation and (307) an holder for inoculated plates; (305) release triggers for the holding of the plates when the piston (501) has raised the plates over the release triggers.

Some of the possible geometrical distributions of the samples into the culture plates, by the proposed device, are represented in fig.3; they are : (10) continuous alternating distribution; (20) discontinuous zone distribution; (30) spiral distribution; (40) discontinuous intermittent distribution.

OPERATION OF THE PROPOSED DEVICE WITH REFERENCE TO FIG. 1 AND FIG.2

The apparatus allows the repetitive execution of all operations concerning the withdrawal and distribution of analytical samples into culture plates.

After housing, into the holder (304), the plates that must be inoculated and loading of samples (which may be urine, waters and others biological of non biological materials) into the mobile disk (201) the operator starts the operations through the control unit (8) which allows the interaction with the operator himself and which is connected to the microcomputer (7). The mobile disk (201) driven by motor (202) brings the first sample into position for withdrawal.

With the motor (106) the mobile head (1) brings the loop (101) over the sample ready for withdrawal; with the combination of the contemporary movements of the motors (102) and (104) a definite amount of material is withdrawn by the loop from the sample.

At the same time the distribution disk (301) driven by motor (302) brings a plate into a useful position, for the distribution of material to be analised, by extracting the same plate from the holder (304). When the plate is correctly positioned, its cap is raised through a lever with a sucking disk (401) operated by motor (403); the cap is hold by the vacuum created by the pump (402).

Immediately after the withdrawal of sample, the activation of motor (106) rotates the mobile head allowing the loop to enter into the opened plate. The combination of the contemporary movements of motors (102), (104), (106) and eventually of motor (306) (the later allows the rotation of the plate) allows the distribution of the material into the plate according to distribution geometries described as example in fig.3 and called : (10) continuous alternating distribution, (20) discontinuous zone distribution, (30) spiral distribution, (40) discontinuous intermittent distribution.

Immediately after the distribution has been completed, the mobile head (1) brings the loop (101) over the sterilisation oven (6) and the combined movement of motors (102) and (104) allow the introduction of the loop into the load coil (601). The activation of the induction oven (6) induces the loop to red hot and its sterilisation. Contemporarily the activation of motor (403) and the disactivation of the pump (402) induces the laying down of the cap for the lock of the plate (303). The distribution disk (301) with its rotation brings the plate, inoculated with material, over the piston (501) which is raised by motor (502). In this way the inoculated plate is raised over the release triggers (305) which do not allow, when crossed by the plate, the fall of the same plate obtaining, in this way, a regular piling.

When piston (501) descends, the operation cycle of the apparatus is finished. All operations are controlled by (7) microcomputer and power electronic circuitry.

**Claims**

1)- Device suitable to carry out, in automatically way, the withdrawal and standardized distribution of analytical samples into plates containing culture media for microbiology.
The device includes : means for withdrawal and distribution of samples to be analysed, including the device suitable to direct contact with the samples: means for positioning the plates that will be inoculated; means for positioning of samples hold into suitable holders; means for opening and closing the plates; means for positioning the plates after their inoculation; means for moving all means above-mentioned; means for the sterilisation of the device suitable for the contact with the material to be analysed; means to control all operations; means of interface with operator.

2)- Device as in claim 1) characterized by the fact that the means for movement may be constituted by electromechanical apparatus, electropneumatic apparatus, hidraulic apparatus, oleodynamic apparatus.

3)- Device as in claim 1) characterized by the fact that the device suitable for direct contact with samples that will be analysed may consist of a calibrated loop of magnetic material, of non magnetic material, of syntetic material, of glass material, of ceramic material.

4)- Device as in claim 1) characterized by the fact that the device suitable for direct contact with the samples that will be analysed may be fixed or replaceable or disposable.

5)- Device as in claim 1) characterized by the fact that the means for positioning the samples in analysis may be removable allowing an easier and rapid loading of samples.

6)- Device as in claim 1) characterized by the fact that it may consist of more than one device for positioning the samples that will be analysed.

7)- Device as in claim 1) characterized by the fact that it may consist of more than one device for positioning the plates suitable for inoculation.

8)- Device as in claim 1) characterized by the fact that the means for sterilisation of the device suitable for direct contact with samples may consist of induction oven, electrical oven, Bunsen burner, device emitting ultraviolet radiation, device for emission of X-ray radiation, device for emission of gamma radiation, container with sterilisation fluid.

9)- Device as in claim 1) characterized by the fact that it allows the distribution of the material to be analysed with selectable geometries and in particular : continuous alternating distribution, discontinuous zone distribution, spiral distribution, intermittent discontinuous distribution.

10)- Device as in claim 1) characterized by the fact that it may execute more than one operation cycle defined as : withdrawal of sample, distribution of sample into the plate, sterilisation of device suitable for direct contact with material in analysis.

11)- Device as in claim 1) characterized by the fact that it may be used in the biomedical field, in the ecological field, in nourishment field, in public health field.

FIG. 1

# FIG. 2

# FIG. 3